# FASCICULE DE BREVET EUROPEEN

(11) **EP 0 542 824 B1**
(45) Date de publication et mention de la délivrance du brevet: **30.08.1995**
(21) Numéro de dépôt: 91914282.8
(22) Date de dépôt: 30.07.1991
(51) Int. Cl.: A61K 9/22, A61K 47/38, A61K 47/42

(54) **PROCEDE DE PREPARATION D'UNE FORME GALENIQUE BIO-ADHESIVE ET FORME GALENIQUE AINSI PREPAREE**
VERFAHREN ZUR HERSTELLUNG EINER BIOADHÄSIVEN ARZNEIFORM SOWIE AUF DIESE WEISE HERGESTELLTE ARZNEIFORM
METHOD FOR PREPARING A BIOADHESIVE GALENICAL AND GALENICAL THEREBY OBTAINED

(30) Priorité: 31.07.1990 FR 9009752
(43) Date de publication de la demande: 26.05.1993
(73) Titulaire: Aiache, Jean-Marc, F-63000 Clermont-Ferrand (FR)
(72) Inventeur: Aiache, Jean-Marc, F-63000 Clermont-Ferrand (FR)
(74) Mandataire: Warcoin, Jacques
(86) Numéro de dépôt international: FR9100630
(87) Numéro de publication internationale: WO9202209

(56) Documents cités:
- EP-A- 0 306 454
- US-A- 3 133 862

## Description

La présente invention se rapporte à une forme galénique bio-adhésive et à son procédé de préparation. Elle se rapporte notamment à une forme galénique à libération controlée, pouvant rester fixée sur toutes les muqueuses, buccale, perlinguale, nasale, vaginale ou rectale pendant une durée prolongée.

L'efficacité thérapeutique d'un médicament est conditionnée par sa biodisponibilité, c'est à dire la proportion de principe actif arrivant dans la circulation générale, et selon quelle cinétique.

L'un des buts des formulations galéniques est donc d'obtenir une forme présentant une bonne biodisponibilité, et d'administration aisée pour le patient.

La voie orale constitue une des voies d'abord privilégiée, par son administration commode et son faible coût. Les médicaments ainsi administrés pourront être absorbés tout au long du tractus digestif. Ils subiront de ce fait différentes biodégradations sous l'action des enzymes, des sucs digestifs, ainsi que des variations de pH.

En outre, après absorption au niveau de l'estomac, du duodénum, ou de l'intestin, les principes actifs seront tout d'abord transportés au foie, où ils pourront être métabolisés, ce qui entraîne le plus souvent une inactivation, au moins partielle, des composés. La cinétique de distribution est de plus, difficile à contrôler.

L'administration par voie parentérale permet d'éviter cet effet de premier passage hépatique. On obtient un effet rapide, mais fugace, qui impose des injections répétées pour maintenir une concentration active. Il y a donc contraintes pour le patient, et risques d'effets secondaires, en raison des concentrations plasmatiques élevées qui sont atteintes rapidement. Dans le cas de substances pouvant entraîner une accoutumance, comme par exemple les opiacés, le risque de dépendance est accru.

Une voie particulièrement intéressante est donc l'administration de produits par la muqueuse buccale, en vue d'une absorption locale à travers l'épithélium. En effet, des conditions favorables à l'absorption médicamenteuse existent au niveau de la cavité buccale :
épithélium pluricellulaire mince, pH faiblement acide et riche vascularisation permettant un passage rapide à travers la muqueuse vers le milieu sanguin. Une concentration sanguine plus élevée que celle obtenue après absorption à un niveau inférieur du tractus gastro-intestinal est souvent observée. Ceci tient au fait que le sang efférent, par les voies maxillaires et sublinguales, se jette dans la veine jugulaire externe, évitant ainsi au principe actif absorbé une bio-transformation rapide par le foie, telle que peuvent subir les molécules absorbées au niveau intestinal. Mais également le principe actif échappe à l'action de la variation de pH des différents niveaux du tube digestif et à celle des enzymes et éventuellement à une complexation avec des substances alimentaires. Il s'agit donc d'une voie alliant la bonne biodisponibilité de la voie parentérale à la facilité d'administration de la voie orale.

L'administration au niveau des autres muqueuses rectales perlinguales, nasales ou vaginales présentera également de bonnes caractéristiques d'absorption, en évitant l'effet de premier passage hépatique. Une action locale, comme par exemple antiseptique, antimycosique, antiinflammatoire, ou astringente, peut également être recherchée, d'où l'intérêt d'une forme apte à rester en place pendant une durée prolongée.

Pour l'administration orale, des formes lyophilisées ont été utilisées. Il s'agit le plus souvent de comprimés pour la voie perlinguale, présentant un délitement rapide lors de la réhydratation par la salive, assurant au principe actif une action rapide mais de courte durée.

Divers problèmes sont posés pour maintenir le plus longtemps possible le principe actif au contact de la muqueuse et pouvoir régler la libération du principe actif à partir de cette forme dans les conditions les plus strictes afin de ne pas provoquer d'absorption massive et de ne pas perturber la vie du patient en lui mettant, dans le cas des formes à absorption buccale, une forme gênante dans la bouche.

De nombreuses formes bioadhésives ont été proposées jusqu'à maintenant, elles étaient toutes basées sur l'utilisation de mélanges de polymères (pectines, dérivés de la cellulose, polycarbophiles) qui sont des polymères visqueux en solution et qui présentent des propriétés d'adhésivité se rapprochant des colles. Les formules réalisées présentent généralement une adhésivité mais qui ne dure guère au-delà d'une heure.

Le brevet US 3 133 862 a décrit des comprimés de vitamine A dépourvus de carbohydrate et contenant un protéinate de sodium.

C'est pourquoi la présente invention se rapporte à un procédé de préparation d'une forme galénique bio-adhésive à libération prolongée, caractérisé en ce que :
a) au moins une partie des principes actifs est mélangée avec une quantité de protéines naturelles représentant au moins 50 % en poids de principe actif, et avec 0,5 à 10% d'un polymère hydrophile,
b) le mélange obtenu à l'issue de l'étape a, subit une granulation en présence d'alcool a environ 60%, puis un séchage pour l'amener à un taux d'humidité d'environ 3%,
c) les grains ainsi obtenus, après calibrage, sont mélangés aux excipients usuels de formulation, en particulier des produits de charge, des lubrifiants, des aromatisants, et des édulcorants,
d) le mélange obtenu à l'issue de l'étape c, subit une compression en vue d'obtenir des comprimés bio-adhésifs à libération prolongée.

On entend par forme bio-adhésive une forme présentant une capacité d'adhésion aux tissus biologiques, en particulier aux muqueuses. Il y a formation de liaisons bio-adhésives entre un élément de la forme galénique, en général un polymère, et le mucus recouvrant les membranes, quand il existe de fortes intéractions entre les deux surfaces. Il peut s'agir de liaisons de nature physique, mécanique ou chimique.

Dans le procédé selon la présente invention, l'élément établissant des liaisons avec les tissus est constitué par des protéines naturelles, n'ayant subi aucune modification. Ces protéines naturelles ne présentent pas de toxicité et ont un prix de revient faible.

Ces protéines sont définies par leurs propriétés fonctionnelles, c'est à dire l'ensemble des propriétés non nutritionnelles influençant leur utilité, à savoir les propriétés physico-chimiques permettant de contribuer a des caractéristiques désirées de la composition dans laquelle elles entrent. Ces propriétés découlent des interactions complexes entre la composition, la structure, la conformation et les propriétés physico-chimiques des protéines entre elles et avec les autres éléments de la composition.

Les propriétés fonctionnelles sont caractéristiques d'une protéine ou d'un mélange de protéines.

Les protéines possèdent une structure macromoléculaire les caractérisant comme un polymère.

Les autres propriétés permettant de les retenir comme excipient principal dans le procédé selon la présente invention sont :
- des propriétés d'hydratation : adsorption, gonflement adhésion, viscosité,
- des propriétés dépendantes des interactions protéines/ protéines, qui interviennent lors de phénomènes tels que la précipitation, la gélification, et la formation de structure,
- des propriétés de surface : elles possèdent des propriétés émulsionnantes et moussantes.

Une expression optimale de ces propriétés, en particulier l'aptitude au gonflement, d'adhésivité et la lenteur de désagrégation due à la formation de structure est obtenue pour des teneurs en protéines naturelles représentant au moins de l'ordre de 50 % en poids du ou des principes actifs utilisés. Cette teneur peut être modulée selon le volume de principe actif utilisé, puisque les protéines jouent également un rôle de liant et de diluant. L'augmentation du pourcentage de protéines permet d'accroître les possibilités de captation d'eau. Le rapport principe actif/protéines dépend de la teneur en principe actif du comprime, elle-même fonction de la dose nécessaire pour obtenir une activité pharmacologique. Ce rapport peut donc varier dans des proportions très importantes.

Une petite quantité d'un polymère hydrophile, de l'ordre de 0,5 à 10%, permet de renforcer ces propriétés fonctionnelles grâce à ses propriétés gélifiantes. La quantité utilisée sera fonction de la vitesse de prise du pouvoir adhésif désirée. On utilisera de préférence une quantité de polymère de l'ordre de 5%. Ce polymère pourra être un polymère organique, par exemple une gomme arabique gomme adragante, gomme guar, caroube, amidon, acide alginique et alginates, carraghénates, agar agar, des dérivés de la cellulose comme la méthyl cellulose, éthylhydroxyéthylcellulose, carboxyméthylcellulose, hydroxypropylméthylcellulose, Avicel RC 581, gomme xanthane ou gélatine.

Selon le type de la proportion utilisée, la vitesse et l'intensité de la bioadhésion peuvent être modulées. De plus le polymère donne aux protéines une meilleure aptitude à la compression et renforce la cohésion du comprimé en favorisant l'hydratation du milieu protéinique.

Selon l'un des aspects préférés de la présente invention, on utilise de l'hydroxypropylméthylcellulose (ou HPMC).

De manière préférée, la quantité de protéines naturelles introduites à l'étape a) représente 50 à 150 % en poids des principes actifs, mais il est bien entendu que l'homme du métier peut augmenter ce pourcentage selon des paramètres qu'il sera à même de déterminer.

Une granulation humide est effectuée en présence d'alcool à environ 60%. Il est important que le titre d'alcool ne soit pas trop élevé, afin de ne pas dénaturer les protéines naturelles et de maintenir leurs propriétés de bio-adhésivité. Un degré alcoolique très supérieur à 60% entraînerait la coupure des ponts intramoléculaires et la modification des propriétés électrophorétiques, se traduisant par une perte de bio-adhésivité.

Selon les conditions de granulation et de séchage (étuve ou lit d'air fluidisé par exemple), on obtient des grains de différentes granulométries. On préférera un séchage à l'étuve à 45°C.

Un calibrage permet de récupérer les grains ayant le diamètre approprié en fonction de la cinétique de libération ultérieure souhaitée. En effet, on constate que la libération du principe actif est ralentie pour des grains ayant un diamètre moyen supérieur à 1 mm.

Les excipients usuels de compression peuvent comprendre des produits de charge et des lubrifiants, comme le talc, l'amidon de maïs, les stéarates. En particulier, une quantité d'amidon de maïs, inférieure ou égale à 5% va renforcer la dureté du comprimé et éviter son délitement précoce. La nature de l'excipient additionné aux protéines permet d'augmenter la force d'adhésion. On observe un renforcement mutuel des propriétés d'adhésivité des différents constituants choisis parmi les protéines, l'amidon et la cellulose, et des propriétés gélifiantes.

Dans le cas de comprimés destinés à la voie buccale, on adjoindra à la composition des aromatisants (par exemple pulvescence menthe) et des édulcorants (comme l'aspartam) destinés à rendre l'administration plus agréable et à masquer éventuellement le goût des principes actifs.

Les grains sont mélangés avec les excipients, en général au mélangeur Turbula®, puis soumis à une compression pour obtenir des comprimés bio-adhésifs, qui libéreront progressivement le principe actif.

Selon un des aspects de l'invention, la totalité des principes actifs est introduite lors de la première étape de la préparation. On observera alors un temps de latence avant le début de la libération des principes actifs.

Selon un autre aspect de l'invention, une quantité résiduelle de principe actif, correspondant à 5 à 10% en poids du total des principes actifs entrant dans la formule, est ajoutée, sous forme non granulée, avant la compression. Dès l'humidification de la forme, cette fraction de principe actif pourra être libéré rapidement et jouera le rôle d'amorce en réduisant le temps de latence de l'activité thérapeutique, alors que des comprimés à double couche ne sont pas envisageables pour conserver les propriétés bio-adhésives.

Les protéines naturelles utilisées peuvent être des protéines extraites du soja.

Les protéines naturelles utilisées peuvent être des protéines du lait, ou concentrés protéiques laitiers.

Les concentrés protéiques de lait sont des poudres obtenues à partir du lait cru pasteurisé à l'exclusion de tout autre ingrédient. Selon les techniques mises en oeuvre, la fraction caséine, les protéines totales de lait ou les protéines du lactosérum peuvent être récupérées. Les techniques d'obtention font appel soit à des procédés purement physiques, filtration sur gel et ultrafiltration, soit à des procédés de thermocoagulation.

Selon la fraction protéique qui est récupérée, on distingue :
- les concentrés protéiques de caseine
   Ils sont obtenus par insolubilisation de la caséine à son point isoélectrique. Le coagulum obtenu est ensuite lavé longuement puis séché conduisant à l'obtention d'une poudre ne refermant que la caséine comme élément protéique. Le coagulum peut aussi être solubilisé par un alcali , après neutralisation des concentrés protéiques solubles, les caséinates sont obtenus.
- les concentrés de protéines totales de lait
   Les protéines totales de lait sont récupérées à partir du lait écrémé après ultrafiltration.
   Cette technique permet de les concentrer sélectivement en les séparant des glucides et des minéraux solubles du lait. Les protéines ainsi recueillies sont à l'état natif, sans dénaturation ni modification importante.
- les concentrés protéiques de lactosérum
   Le lactosérum ou petit lait est le sous produit de la fabrication des fromages et de la caséine. Après coagulation enzymatique pour la fabrication des fromages, le lactosérum est le résidu liquide de couleur jaune vert obtenu par séparation. Il peut être assimilé à une solution diluée de sels minéraux, de lactose et de protéines de grande qualité nutritionnelle. La concentration du lactosérum est réalisée par ultrafiltration, chromatographie par échange d'ions ou par précipitation thermique.

Quelle que soit la technique utilisée, on aboutit à une poudre blanche à jaunâtre, plus ou moins agglutinée, à odeur caractéristique de lait, renfermant, outre une fraction protéique, du lactose, des matières grasses et des sels minéraux en quantités définies et variables selon le type de produit. Parmi les minéraux, le calcium et le phosphore sont les plus importants du lait. Lors de la concentration par ultrafiltration, ces minéraux sont concentrés avec les protéines.

On peut utiliser un seul type de protéines, ou un mélange. Les meilleurs résultats sont obtenus avec les concentrés des protéines totales du lait, qui permettent de réaliser sans difficulté une granulation et une compression.

L'utilisation de protéines de lait rend inutile l'addition de lactose avant la compression.

On peut utiliser un mélange de concentrés protéiques de lactosérum en faible pourcentage avec des concentrés de protéines totales.

Selon un des aspects préférés de l'invention, on utilise du Prosobel, en particulier du Prosobel L85® qui donne un comprimé possédant un temps de désagrégation lent (inférieur à celui obtenu par exemple avec du Prosobel L60®), et un gonflement important.

La mise en oeuvre de ce procédé permet d'obtenir une forme galénique présentant des propriétés durables d'adhésivité aux muqueuses, caractérisée en ce qu'elle comporte outre les principes actifs, des protéines naturelles à une teneur représentant au moins 50% en poids des principes actifs, un polymère hydrophile, et des excipients usuels permettant la stabilisation de la formulation.

De préférence, la teneur en protéines naturelles est comprise entre environ 50 % et 150 % en poids des principes actifs.

En effet les protéines, en particulier les concentrés protéiques de lait, forment un réseau après granulation humide et compression permettant l'obtention d'une forme dont le délitement et le comportement sont fonction de la nature et de la proportion des protéines mises en jeu. En présence d'eau, les protéines forment un gel et gonflent progressivement. La couche supérieure du comprimé s'hydrate rapidement et les interactions qui en découlent forment une barrière gélifiée s'opposant à la pénétration de l'eau, et permettant l'établissement de liaisons entre les protéines et la muqueuse, par exemple des liaisons de type Van der Waals.

Cette bioadhésivité, instantanée, résulte de l'hydratation du polymère entraînant la séparation des longues chaînes, les molécules du polymère pouvant alors s'apparier,
- avec les molécules de la muqueuse (glycoprotéines),
- avec d'autres molécules du polymère lui même pour former des liaisons cohésives.

La forme galénique peut se présenter sous forme de comprimes ronds d'environ 8 mm de diamètre et 2 à 3 mm d'épaisseur.

Elle peut également se présenter sous forme de comprimés hémi-convexes qui épousent la forme du sillon maxillo-gingival ou encore de la cavité qui est située sous la langue.

En effet, selon l'un des aspects de l'invention, cette forme galénique est caractérisée en ce qu'elle est apte à rester appliquée sur la muqueuse buccale pendant au moins 10 mn, et pour une durée qui peut atteindre 12 heures.

Pour une administration au niveau de la muqueuse buccale, le comprimé peut être mis en le tenant sur le bout du doigt entre la lèvre inférieure et la gencive, en lui appliquant une légère force manuelle. Après un temps de latence assez court, le comprimé se colle et ne bouge plus. Il est possible de parler et même de s'alimenter sans inconvénient.

L'invention fournit donc une forme de fabrication facile et peu coûteuse, permettant d'éviter l'effet de premier passage hépatique. Elle évite les prises répétées, puisqu'il s'agit d'une forme à libération prolongée bioadhésive. Elle préserve l'indépendance et l'autonomie du malade, d'où un intérêt psychologique évident, puisqu'elle peut être prescrite en dehors du milieu hospitalier. Les effets secondaires sont réduits.

Dans le cas de principes actifs morphiniques, l'absorption qui s'effectue également en faible partie au niveau du tractus digestif à cause de la sécrétion salivaire, produit des métabolites comme la 6-glucuronide-morphine, qui est 1,5 fois plus puissant.

Selon un autre aspect de l'invention, la forme galénique est caractérisée en ce qu'elle est apte à rester appliquée sur la muqueuse perlinguale, nasale, rectale ou vaginale, pour une durée au moins égale à 10 mn, et pouvant atteindre 12 heures.

Les exemples qui suivent sont destinés à illustrer l'invention sans aucunement en limiter la portée.

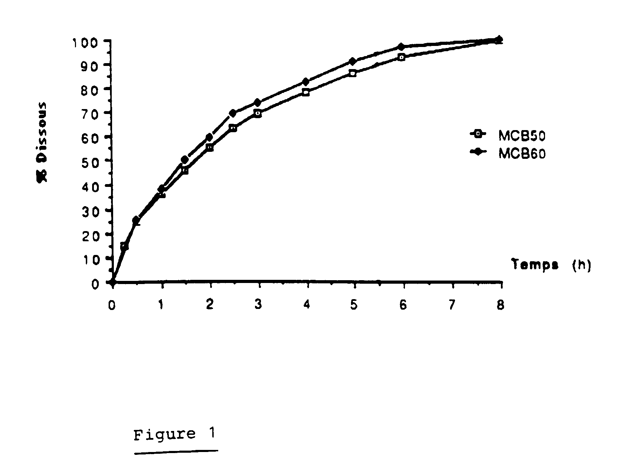

La Figure 1 représente les cinétiques des pourcentages dissous à la palette pour des comprimés dosés à 50 mg (MCB50) et 60 mg (MCB60) de sulfate de morphine.

### EXEMPLE 1 : PREPARATION DE COMPRIMES MUCO ADHESIFS DOSES A 50 mg DE MORPHINE

| **Morphine (50mg)** | | |
|---|---|---|
| - Principe actif : morphine sulfate | 50,00 mg | 38,46 % |
| - Prosobel L85® | 55,00 mg | 42,30 % |
| - HPMC 15 000 | 6,50 mg | 5,00 % |
| - Amidon de maïs | 6,50 mg | 5,00 % |
| - Arôme pulvescence menthe | 5,00 mg | 3,84 % |
| - Aspartam | 5,00 mg | 3,84 % |
| - Talc | 1,00 mg | 0,76 % |
| - Stéarate de magnésium | 1,00 mg | 0,76 % |
| | 1̅3̅0̅,̅0̅0̅ m̅g̅ | |

Les excipients comprenant la totalité des protéines entrant dans la composition plus la moitié du pourcentage de l'arôme et de l'édulcorant sont tamisés sur un tamis de 0,8 mm. Ils sont ensuite mélangés avec le principe actif, au mélangeur Turbula®, pendant 10 minutes. Le mélange subit un mouillage au mélangeur planétaire, avec une solution alcoolique à 60%. Puis on procède à une granulation sur un granulateur oscillant, ayant une grille de 1,6 mm. Le grain obtenu est séché à l'étuve à 45°C pendant 8 heures, de manière à obtenir un taux d'humidité d'environ 3%. Après calibrage sur une grille de 1 mm, le grain obtenu est mélangé avec les lubrifiants et le reste de l'arôme et de l'édulcorant, pendant 10 minutes au turbula. La compression est réalisée sur une machine rotative de type Frogerais, avec des poinçons de 8 mm de diamètre. L'épaisseur des comprimés est de 2 mm.

### EXEMPLE 2 : PREPARATION DE COMPRIMES DE MORPHINE DOSES A 60 mg

| **Morphine (60 mg)** | | |
|---|---|---|
| - Principe actif : morphine sulfate | 60,00 mg | 42,85 % |
| - Prosobel L85® | 55,00 mg | 39,28 % |
| - HPMC 15 000 | 6,50 mg | 4,61 % |
| - Amidon de maïs | 6,50 mg | 4,61 % |
| - Aspartam | 5,00 mg | 3,57 % |
| - Arôme pulvescence menthe | 5,00 mg | 3,57 % |
| - Talc | 1,00 mg | 0,71 % |
| - Stéarate de magnésium | 1,00 mg | 0,71 % |
| | 1̅4̅0̅,̅0̅0̅ m̅g̅ | |

Les comprimés sont préparés selon un protocole analogue a celui de l'exemple 1.

### EXEMPLE 3 :

| **Buprénorphine** | | |
|---|---|---|
| - Buprénorphine | 0,30 mg | 0,21 % |
| - Lactose Codex | 39,70 mg | 28,36 % |
| - HPMC 15 000 | 13,00 mg | 9,29 % |
| - Amidon de maïs | 13,00 mg | 9,29 % |
| - Prosobel L85® | 62,00 mg | 44,29 % |
| - Arôme pulvescence menthe | 5,00 mg | 3,57 % |
| - Aspartam | 5,00 mg | 3,57 % |
| - Talc | 1,00 mg | 0,71 % |
| - Stéarate de magnésium | 1,00 mg | 0,71 % |
| | 1̅4̅0̅,̅0̅0̅ m̅g̅ | |

Les comprimés sont préparés selon un protocole analogue à celui de l'exemple 1.

### EXEMPLE 4 : PREPARATION DE COMPRIMES AVEC UN PRINCIPE ACTIF ANTISPASMODIQUE

| **Phloroglucinol** | | |
|---|---|---|
| - Principe actif : phloroglucinol | 80,00 mg | 50,00 % |
| - HPMC 15 000 | 6,50 mg | 4,10 % |
| - Amidon de maïs | 6,50 mg | 4,10 % |
| - Prosobel L85® | 55,00 mg | 34,37 % |
| - Arôme pulvescence menthe | 5,00 mg | 3,12 % |
| - Aspartam | 5,00 mg | 3,12 % |
| - Stéarate de magnésium | 1,00 mg | 0,62 % |
| - Talc | 1,00 mg | 0,62 % |
| | 1̅6̅0̅,̅0̅0̅ m̅g̅ | |

Les comprimés sont préparés selon un protocole analogue à celui de l'exemple 1.

### EXEMPLE 5 : PREPARATION DE COMPRIMES CONTENANT UN PRINCIPE ACTIF ANTIASTHMATIQUE

| **Salbutamol à 4 mg** | | |
|---|---|---|
| - Principe actif : salbutamol sulfate | 4,00 mg | 2,86 % |
| - Lactose Codex | 56,00 mg | 40,00 % |
| - HPMC 15 000 | 6,50 mg | 4,64 % |
| - Amidon de maïs | 6,50 mg | 4,64 % |
| - Prosobel L85® | 55,00 mg | 39,28 % |
| - Arôme pulvescence menthe | 5,00 mg | 3,57 % |
| - Aspartam | 5,00 mg | 3,57 % |
| - Talc | 1,00 mg | 0,71 % |
| - Stéarate de magnésium | 1,00 mg | 0,71 % |
| | 1̅4̅0̅,̅0̅0̅ m̅g̅ | |

| **Salbutamol à 8 mg** | | |
|---|---|---|
| - Principe actif : salbutamol sulfate | 8,00 mg | 9,09 % |
| - HPMC 15 000 | 6,50 mg | 7,38 % |
| - Amidon de maïs | 6,50 mg | 7,38 % |
| - Prosobel L85® | 55,00 mg | 62,50 % |
| - Arôme pulvescence menthe | 5,00 mg | 5,68 % |
| - Aspartam | 5,00 mg | 5,68 % |
| - Talc | 1,00 mg | 1,13 % |
| - Stéarate de magnésium | 1,00 mg | 1,13 % |
| | 8̅8̅,̅0̅0̅ m̅g̅ | |

Les comprimés sont préparés selon un protocole analogue à celui de l'exemple 1.

### EXEMPLE 6 : LIBERATION DU PRINCIPE ACTIF IN VITRO

Les comprimés obtenus possèdent un poids moyen de 0,132 g, une dureté voisine de 0,61 Newton, et un temps de délitement supérieur à 4 heures. La cinétique des pourcentages dissous pour des comprimés dosés à respectivement à 50 et à 60 mg de morphine est représentée sur la figure 1. Cette cinétique est réalisée sur l'appareil de dissolution à palettes tournantes (type Pharmacopée) à un pH de 4,5 et à 60 tours par minute. Après dosage des prélèvements en HPLC, on constate que le pourcentage dissous au bout de 8 heures est de 100 % dans les deux cas.

### EXEMPLE 7 : RESULTATS DES ESSAIS CLINIQUES ET PHARMACO CINETIQUES

Ces essais ont été réalisés en utilisant des comprimés préparés selon l'exemple 1.

### * Etudes cliniques :

Des essais cliniques préliminaires ont été réalisés sur des malades post-opérés portant sur l'interrogatoire du malade, sur l'activité, la rapidité et la durée de l'action, enfin sur l'acceptabilité de la forme.

Ces études ont montré que la forme était bien acceptée car le goût était noté agréable, surprenant et nouveau par rapport aux médicaments classiques.

L'analgésie débutait environ 1/4 heure après la prise du médicament et durait 8 heures. Cette forme paraissait être bien tolérée par les malades et aucun effet secondaire sévère n'a été révélé.

### * Etudes pharmacocinétique :

Réalisée sur des malades, elle donne les taux plasmatiques suivants. Une échelle visuelle de la douleur établie par les malades montre que l'analgésie peut durer jusqu'à 18 h après la prise du médicament.

| **Taux plasmatiques** | | | |
|---|---|---|---|
| N° sujet | Dose administrée | Tmax (h) | Cmax (ng/ml) |
| 1 | 50 mg | 4 | 24,10 |
| 2 | 50 mg | 3,75 | 11,31 |
| 3 | 50 mg | 2 | 34,97 |
| 4 | 50 mg | 4 | 27,9 |
| 5 | 30 mg | 2,66 | 9,10 |
| 6 | 50 mg | 4 | 20,00 |
| 7 | 2 x 30 mg | 4 | 20,04 |
| 8 | 30 mg | 4 | 4,45 |
| 9 | 30 mg | 4 | 8,21 |
| 10 | 50 mg | 2 | 5,39 |
| Aucun effet secondaire sévère n'a été révélé. | | | |

## Revendications (Revendications pour l'(les) Etat(s) contractant(s) suivant(s): AT, BE, CH, LI, DE, DK, FR, GB, IT, LU, NL, SE)

1. Procédé de préparation d'une forme galénique bio-adhésive à libération prolongée, caractérisé en ce que :
a) au moins une partie des principes actifs est mélangée avec une quantité de protéines naturelles représentant au moins 50 % en poids de principe actif, et avec 0,5 à 10% d'un polymère hydrophile,
b) le mélange obtenu à l'issue de l'étape a, subit une granulation en présence d'alcool à environ 60%, puis un séchage pour l'amener à un taux d'humidité d'environ 3%,
c) les grains ainsi obtenus, après calibrage, sont mélangés aux excipients usuels de formulation, en particulier des produits de charge, des lubrifiants, des aromatisants, et des édulcorants,
d) le mélange obtenu à l'issue de l'étape c, subit une compression en vue d'obtenir des comprimés bio-adhésifs à libération prolongée.

2. Procédé de préparation d'une forme galénique selon la revendication 1, caractérisé en ce que la totalité des principes actifs est introduite à l'étape a.

3. Procédé de préparation d'une forme galénique selon la revendication 1, caractérisé en ce que, on ajoute une quantité résiduelle de principe actif, correspondant à 5 à 10% du poids de principe actif total, sous forme non granulée, au cours de l'étape c.

4. Procédé de préparation d'une forme galénique selon l'une des revendications 1 à 3, caractérisé en ce que la quantité de protéines naturelles introduite à l'étape a) représente 50 à 150 % en poids du principe actif.

5. Procédé de préparation d'une forme galénique selon l'une des revendications 1 à 4, caractérisé en ce que, les produits de charge comprennent de l'amidon de maïs.

6. Procédé de préparation d'une forme galénique selon l'une des revendications 1 à 5, caractérisé en ce que le polymère hydrophile est un polymère organique dérivé de cellulose, en particulier de l'hydroxypropylméthylcellulose.

7. Procédé de préparation d'une forme galénique selon l'une des revendications 1 à 6, caractérise en ce que les protéines naturelles sont des protéines de soja.

8. Procédé de préparation d'une forme galénique selon l'une des revendications 1 à 6, caractérise en ce que les protéines naturelles sont des protéines du lait.

9. Procédé de préparation d'une forme galénique selon la revendication 8, caractérisé en ce que les protéines naturelles sont du Prosobel L85^{R}.

10. Forme galénique à libération prolongée, présentant des propriétés durables d'adhésivité aux muqueuses, susceptible d'être obtenue par la mise en oeuvre du procédé selon l'une des revendications 1 à 9, caractérisée en ce qu'elle comporte outre les principes actifs, des protéines naturelles à une teneur représentant au moins 50% en poids des principes actifs, un polymère hydrophile, et des excipients usuels permettant la stabilisation de la formulation.

11. Forme galénique selon la revendication 10, caractérisée en ce que la teneur en protéines naturelles est comprise entre 50 % et 150 % en poids des principes actifs.

12. Forme galénique selon l'une des revendications 10 et 11, caractérisée en ce qu'il s'agit d'un comprimé d'environ 8 mm de diamètre et de 2 à 3 mm d'épaisseur.

13. Forme galénique selon l'une des revendications 10 à 12, caractérisée en ce qu'elle présente une conformation adaptée à l'application sur la muqueuse buccale, perlinguale, nasale, rectale et/ou vaginale.

## Revendications (Revendications pour l'(les) Etat(s) contractant(s) suivant(s): ES, GR)

1. Procédé de préparation d'une forme galénique bio-adhésive à libération prolongée, caractérisé en ce que :
a) au moins une partie des principes actifs est mélangée avec une quantité de protéines naturelles représentant au moins 50 % en poids de principe actif, et avec 0,5 à 10% d'un polymère hydrophile,
b) le mélange obtenu à l'issue de l'étape a, subit une granulation en présence d'alcool à environ 60%, puis un séchage pour l'amener à un taux d'humidité d'environ 3%,
c) les grains ainsi obtenus, après calibrage, sont mélangés aux excipients usuels de formulation, en particulier des produits de charge, des lubrifiants, des aromatisants, et des édulcorants,
d) le mélange obtenu à l'issue de l'étape c, subit une compression en vue d'obtenir des comprimés bio-adhésifs à libération prolongée.

2. Procédé de préparation d'une forme galénique selon la revendication 1, caractérisé en ce que la totalité des principes actifs est introduite à l'étape a.

3. Procédé de préparation d'une forme galénique selon la revendication 1, caractérisé en ce que, on ajoute une quantité résiduelle de principe actif, correspondant à 3 à 10% du poids de principe actif total, sous forme non granulée, au cours de l'étape c.

4. Procédé de préparation d'une forme galénique selon l'une des revendications 1 à 3, caractérisé en ce que la quantité de protéines naturelles introduite à l'étape a) représente 50 à 150 % en poids du principe actif.

5. Procédé de préparation d'une forme galénique selon l'une des revendications 1 à 4, caractérisé en ce que, les produits de charge comprennent de l'amidon de maïs.

6. Procédé de préparation d'une forme galénique selon l'une des revendications 1 à 5, caractérisé en ce que le polymère hydrophile est un polymère organique dérivé de cellulose, en particulier de l'hydroxypropylméthylcellulose.

7. Procédé de préparation d'une forme galénique selon l'une des revendications 1 à 6, caractérisé en ce que les protéines naturelles sont des protéines de soja.

8. Procédé de préparation d'une forme galénique selon l'une des revendications 1 à 6, caractérisé en ce que les protéines naturelles sont des protéines du lait.

9. Procédé de préparation d'une forme galénique selon la revendication 8, caractérisé en ce que les protéines naturelles sont du Prosobel L85^{R} .

## Claims (Claims for the following Contracting State(s): AT, BE, CH, LI, DE, DK, FR, GB, IT, LU, NL, SE)

1. Process for preparing a bioadhesive sustained-release pharmaceutical dosage form, characterized in that:
a) at least a part of the active principles is mixed with a quantity of natural proteins representing at least 50 % by weight of active principle, and with 0.5 to 10 % of a hydrophilic polymer,
b) the mixture obtained at the end of step a is subjected to granulation in the presence of approximately 60% alcohol, followed by drying to bring it to a moisture content of approximately 3 %.
c) the granules thereby obtained, after sizing, are mixed with standard formulation excipients, especially ballast materials, lubricants, flavorings and sweeteners,
d) the mixture obtained at the end of step c, is subjected to tabletting for the purpose of obtaining bioadhesive sustained-release tablets.

2. Process for preparing a pharmaceutical dosage form according to Claim 1, characterized in that the active principles are introduced in their entirety in step a.

3. Process for preparing a pharmaceutical dosage form according to Claim 1, characterized in that a residual quantity of active principle, corresponding to 5 to 10 % of the total weight of active principle, is added in nongranular form during step c.

4. Process for preparing a pharmaceutical dosage form according to one of Claims 1 to 3, characterized in that the quantity of natural proteins introduced in step a) represents 50 to 150 % by weight of the active principle.

5. Process for preparing a pharmaceutical dosage form according to one of Claims 1 to 4, characterized in that the ballast materials comprise corn starch.

6. Process for preparing a pharmaceutical dosage form according to one of Claims 1 to 5, characterized in that the hydrophilic polymer is an organic polymer derived from cellulose, especially hydroxypropyl methyl-cellulose.

7. Process for preparing a pharmaceutical dosage form according to one of Claims 1 to 6, characterized in that the natural proteins are soya bean proteins.

8. Process for preparing a pharmaceutical dosage form according to one of Claims 1 to 6, characterized in that the natural proteins are milk proteins.

9. Process for preparing a pharmaceutical dosage form according to Claim 8, characterized in that the natural proteins are Prosobel L85®.

10. Sustained-release pharmaceutical dosage form displaying long-lasting properties of adhesiveness to the mucosae, capable of being obtained by carrying out the process according to one of Claims 1 to 9, characterized in that it contains, apart from the active principles, natural proteins in an amount representing at least 50 % by weight of the active principles, a hydrophilic polymer, and standard excipients enabling the formulation to be stabilized.

11. Pharmaceutical dosage form according to Claim 10, characterized in that the content of natural proteins is between 50 % and 150 % by weight of the active principles.

12. Pharmaceutical dosage form according to one of Claims 10 and 11, characterized in that it is a tablet approximately 8 mm in diameter and from 2 to 3 mm in thickness.

13. Pharmaceutical dosage form according to one of Claims 10 to 12, characterized in that it has a shape suitable for application to the buccal, perlingual, nasal, rectal and/or vaginal mucosa.

## Claims (Claims for the following Contracting State(s): ES, GR)

1. Process for preparing a bioadhesive sustained-release pharmaceutical dosage form, characterized in that:
a) at least a part of the active principles is mixed with a quantity of natural proteins representing at least 50 % by weight of active principle, and with 0.5 to 10 % of a hydrophilic polymer,
b) the mixture obtained at the end of step a is subjected to granulation in the presence of approximately 60% alcohol, followed by drying to bring it to a moisture content of approximately 3 %.
c) the granules thereby obtained, after sizing, are mixed with standard formulation excipients, especially ballast materials, lubricants, flavorings and sweeteners,
d) the mixture obtained at the end of step c, is subjected to tabletting for the purpose of obtaining bioadhesive sustained-release tablets.

2. Process for preparing a pharmaceutical dosage form according to Claim 1, characterized in that the active principles are introduced in their entirety in step a.

3. Process for preparing a pharmaceutical dosage form according to Claim 1, characterized in that a residual quantity of active principle, corresponding to 5 to 10 % of the total weight of active principle, is added in nongranular form during step c.

4. Process for preparing a pharmaceutical dosage form according to one of Claims 1 to 3, characterized in that the quantity of natural proteins introduced in step a) represents 50 to 150 % by weight of the active principle.

5. Process for preparing a pharmaceutical dosage form according to one of Claims 1 to 4, characterized in that the ballast materials comprise corn starch.

6. Process for preparing a pharmaceutical dosage form according to one of Claims 1 to 5, characterized in that the hydrophilic polymer is an organic polymer derived from cellulose, especially hydroxypropyl methyl-cellulose.

7. Process for preparing a pharmaceutical dosage form according to one of Claims 1 to 6, characterized in that the natural proteins are soya bean proteins.

8. Process for preparing a pharmaceutical dosage form according to one of Claims 1 to 6, characterized in that the natural proteins are milk proteins.

9. Process for preparing a pharmaceutical dosage form according to Claim 8, characterized in that the natural proteins are Prosobel L85®.

## Patentansprüche (Patentansprüche für folgende(n) Vertragsstaat(en): AT, BE, CH, LI, DE, DK, FR, GB, IT, LU, NL, SE)

1. Verfahren zur Herstellung eines bio-haftenden galenischen Präparats mit verlängerter Freisetzung (Depotwirkung), dadurch gekennzeichnet, daß
a) mindestens ein Teil dar Wirkstoffe mit einer Menge von natürlichen Proteinen, die mindestens 50 Gew.-% der Wirkstoffe ausmacht, und mit 0,5 bis 10 % eines hydrophilen Polymers gemischt wird,
b) die in dar Stufe (a) erhaltene Mischung in Gegenwart von etwa 60 %igem Alkohol granuliert und dann getrocknet wird, um ihren Feuchtigkeitsgehalt auf etwa 3 % zu bringen,
c) die so erhaltenen Körnchen nach dem Klassieren mit üblichen Formulierungs-Exzipienten, insbesondere Füllstoffen, Gleitmitteln, Aromatisierungsmitteln und Süßungsmitteln, gemischt werden und
d) die in der Stufe (c) erhaltene Mischung gepreßt wird zur Herstellung von bio-haftenden Tabletten mit verlängerter Freisetzung (Depotwirkung).

2. Verfahren zur Herstellung eines galenischen Präparats nach Anspruch 1, dadurch gekennzeichnet, daß die Gesamtmenge der Wirkstoffe in der Stufe (a) eingeführt wird.

3. Verfahren zur Herstellung eines galenischen Präparats nach Anspruch 1, dadurch gekennzeichnet, daß man eine Restmenge an Wirkstoff, die 5 bis 10 Gew.-% der Gesamtmenge des Wirkstoffes entspricht, in nicht-granulierter Form während der Stufe (c) zugibt.

4. Verfahren zur Herstellung eines galenischen Präparats nach einem der Ansprüche 1 bis 3, dadurch gekennzeichnet, daß die Menge der in der Stufe (a) eingeführten natürlichen Proteine 50 bis 150 Gew.-% des Wirkstoffes beträgt.

5. Verfahren zur Herstellung eines galenischen Präparats nach einem der Ansprüche 1 bis 4, dadurch gekennzeichnet, daß die Füllstoffe Maisstärke umfassen.

6. Verfahren zur Herstellung eines galenischen Präparats nach einem der Ansprüche 1 bis 5, dadurch gekennzeichnet, daß es sich bei dem hydrophilen Polymer um ein organisches Polymer handelt, das von Cellulose, insbesondere Hydroxypropylmethylcellulose, abgeleitet ist.

7. Verfahren zur Herstellung eines galenischen Präparats nach einem der Ansprüche 1 bis 6, dadurch gekennzeichnet, daß es sich bei den natürlichen Proteinen um Sojaproteine handelt.

8. Verfahren zur Herstellung eines galenischen Präparats nach einem der Ansprüche 1 bis 6, dadurch gekennzeichnet, daß es sich bei den natürlichen Proteinen um Milchproteine handelt.

9. Verfahren zur Herstellung eines galenischen Präparats nach Anspruch 8, dadurch gekennzeichnet, daß es sich bei den natürlichen Proteinen um Prosobel L85® handelt.

10. Galenisches Präparat mit verlängerter Freisetzung (Depotwirkung), das dauerhafte Haftungseigenschaften an den Schleimhäuten aufweist und erhältlich ist durch Durchfilhrung des Verfahrens nach einem der Ansprüche 1 bis 9, dadurch gekennzeichnet, daß es außer den Wirkstoffen natürliche Proteine in einem Gehalt von mindestens 50 Gew.-% der Wirkstoffe, ein hydrophiles Polymer und übliche Exzipienten, welche die Stabilisierung des Präparats erlauben, enthält.

11. Galenisches Präparat nach Anspruch 10, dadurch gekennzeichnet, daß sein Gehalt an natürlichen Proteinen zwischen 50 und 150 Gew.-% der Wirkstoffe liegt.

12. Galenisches Präparat nach einem der Ansprüche 10 und 11, dadurch gekennzeichnet, daß es sich dabei um eine Tablette mit einem Durchmesser von etwa 8 mm und einer Dicke von etwa 2 bis 3 mm handelt.

13. Galenisches Präparat nach einem der Ansprüche 10 bis 12, dadurch gekennzeichnet, daß es eine Form hat, die geeignet ist für die Applikation auf die Mund-, Zungen-, Nasen-, Rektal- und/oder Vaginal-Schleimhaut.

## Patentansprüche (Patentansprüche für folgende(n) Vertragsstaat(en): ES, GR)

1. Verfahren zur Herstellung eines bio-haftenden galenischen Präparats mit verlängerter Freisetzung (Depotwirkung), dadurch gekennzeichnet, daß
a) mindestens ein Teil der Wirkstoffe mit einer Menge von natürlichen Proteinen, die mindestens 50 Gew.-% der Wirkstoffe ausmacht, und mit 0,5 bis 10 % eines hydrophilen Polymers gemischt wird,
b) die in der Stufe (a) erhaltene Mischung in Gegenwart von etwa 60 %igem Alkohol granuliert und dann getrocknet wird, um ihren Feuchtigkeitsgehalt auf etwa 3 % zu bringen,
c) die so erhaltenen Körnchen nach dem Klassieren mit üblichen Formulierungs-Exzipienten, insbesondere Füllstoffen, Gleitmitteln, Aromatisierungsmitteln und Süßungsmitteln,gemischt werden und
d) die in der Stufe (c) erhaltene Mischung gepreßt wird zur Herstellung von bio-haftenden Tabletten mit verlängerter Freisetzung (Depotwirkung).

2. Verfahren zur Herstellung eines galenischen Präparats nach Anspruch 1, dadurch gekennzeichnet, daß die Gesamtmenge der Wirkstoffe in der Stufe (a) eingeführt wird.

3. Verfahren zur Herstellung eines galenischen Präparats nach Anspruch 1, dadurch gekennzeichnet, daß man eine Restmenge an Wirkstoff, die 5 bis 10 Gew.-% der Gesamtmenge des Wirkstoffes entspricht, in nicht-granulierter Form während der Stufe (c) zugibt.

4. Verfahren zur Herstellung eines galenischen Präparats nach einem der Ansprüche 1 bis 3, dadurch gekennzeichnet, daß die Menge der in der Stufe (a) eingeführten natürlichen Proteine 50 bis 150 Gew.-% des Wirkstoffes beträgt.

5. Verfahren zur Herstellung eines galenischen Präparats nach einem der Ansprüche 1 bis 4, dadurch gekennzeichnet, daß die Füllstoffe Maisstärke umfassen.

6. Verfahren zur Herstellung eines galenischen Präparats nach einem der Ansprüche 1 bis 5, dadurch gekennzeichnet, daß es sich bei dem hydrophilen Polymer um ein organisches Polymer handelt, das von Cellulose, insbesondere Hydroxypropylmethylcellulose, abgeleitet ist.

7. Verfahren zur Herstellung eines galenischen Präparats nach einem der Ansprüche 1 bis 6, dadurch gekennzeichnet, daß es sich bei den natürlichen Proteinen um Sojaproteine handelt.

8. Verfahren zur Herstellung eines galenischen Präparats nach einem der Ansprüche 1 bis 6, dadurch gekennzeichnet, daß es sich bei den natürlichen Proteinen um Milchproteine handelt.

9. Verfahren zur Herstellung eines galenischen Präparats nach Anspruch 8, dadurch gekennzeichnet, daß es sich bei den natürlichen Proteinen um Prosobel L85® handelt.
